# EUROPEAN PATENT APPLICATION

(11) **EP 0 544 240 A1**
(43) Date of publication of application: **02.06.1993**
(21) Application number: 92120025.9
(22) Date of filing: 25.11.1992
(51) Int. Cl.: C07D 241/38, A61K 31/495

(54) **Benzoquinoxalines and pharmaceutical compositions containing them**

(30) Priority: 28.11.1991 DE 4139143
(71) Applicant: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Gull, Peter, CH-4138 Pfeffingen (CH); Markstein, Rudolf, W-7888 Rheinfelden (DE); Swoboda, Robert, CH-3098 Köniz (CH)

(57) **Abstract**

6-hydroxy or 6-alkoxy-1,2,3,4,4a,5,10,10a-octahydro-benzo[g]quinoxalines bearing in position 4 an optionally substituted phenyl group may be used in the treatment of schizophrenia, drug abuse or self-injurious behaviour.

## Description

The present invention relates to benzoquinoxalines, their production, their use as pharmaceuticals and pharmaceutical compositions containing them.

In particular the present invention provides 6-hydroxy or 6-alkoxy-1,2,3,4,4a,5,10,10a-octahydro-benzo[g]quinoxalines bearing in position 4 an optionally substituted phenyl group, in free base or acid addition salt form.

More particularly the present invention provides compounds of formula I 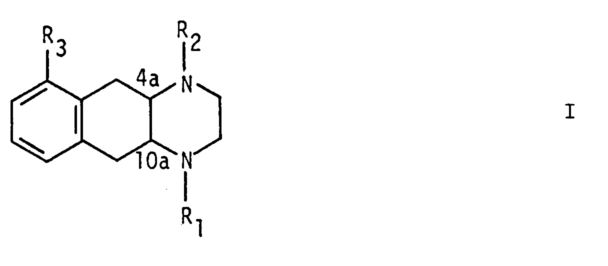
wherein
- R₁: is (C₁₋₄)alkyl,
- R₂: is phenyl optionally mono-, di- or trisubstituted by halogen with an atomic number of 9 to 53, (C₁₋₄)alkyl and/or (C₁₋₄)alkoxy, and
- R₃: is hydroxy or (C₁₋₄)alkoxy,
in free base or acid addition salt form.

The compounds of formula I present two asymmetrical carbon atoms in positions 4a and 10a. They may therefore appear in racemic or optically active forms. The invention relates to all optical isomers and their mixtures including the racemic mixtures.

In positions 4a and 10a the compounds of formula I may have the cis configuration or the trans configuration. The compounds with the trans configuration are preferred. These include compounds with the configuration IA as well as compounds with the configuration IB 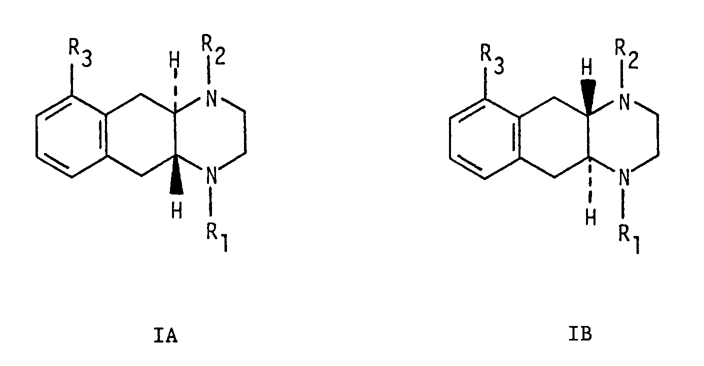
and the corresponding racemates.

The alkyl and alkoxy groups contained in the compounds of formula I preferably contain 1 or 2 carbon atoms, and in particular represent methyl or methoxy.

Halogen as defined above is preferably chlorine, bromine or fluorine.
R₂ preferably is a group of formula (a) 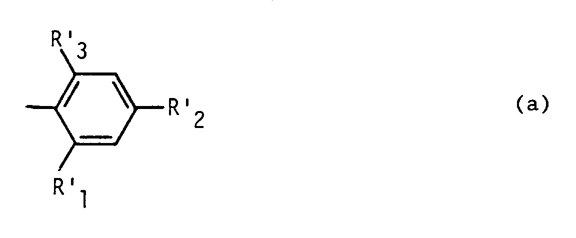
wherein
- R'₁: is hydrogen or (C₁₋₄)alkyl,
- R'₂: is hydrogen, halogen with an atomic number of 9 to 53 or (C₁₋₄)alkyl, and
- R'₃: is hydrogen or (C₁₋₄)alkyl.

In a group of compounds of formula I, R₁ is (C₁₋₄)alkyl, R₂ is a group of formula (a) and R₃ is hydroxy or (C₁₋₄)alkoxy.

In accordance with the invention, the compounds of formula I and their salts are obtained, whereby
a) in order to produce the compounds of formula Ia, 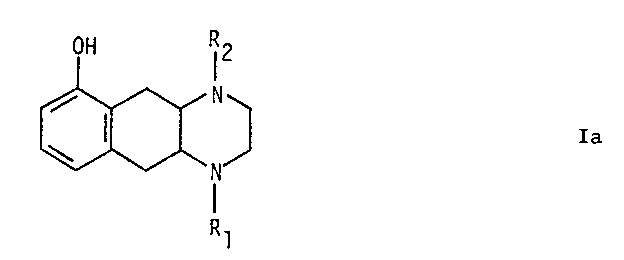 wherein R₁ and R₂ are defined as above, the ether group in compounds of formula Ib, 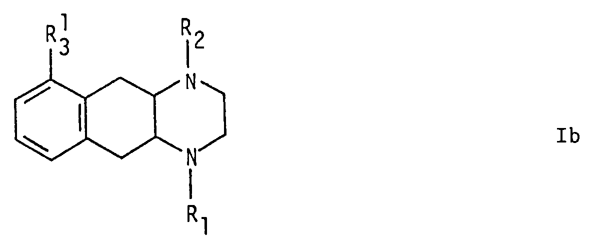 wherein R₁ and R₂ are defined as above, and R¹₃ is alkoxy with 1 to 4 carbon atoms, is cleaved, or
b) in order to produce compounds of formula Ib, compounds of formula II, 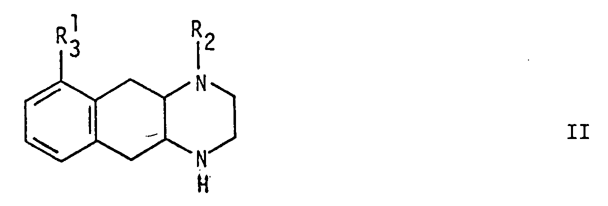 wherein R₂ and R¹₃ are defined as above, are alkylated, or
c) in order to produce compounds of formula I'b, 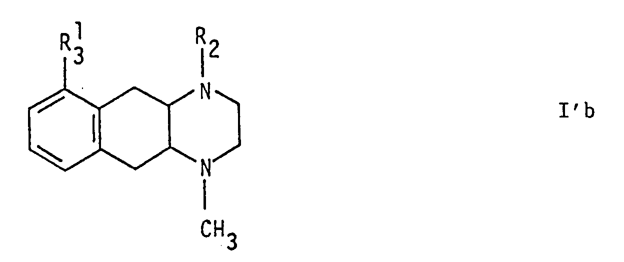 wherein R₂ and R¹₃ are defined as above, compounds of formula III, 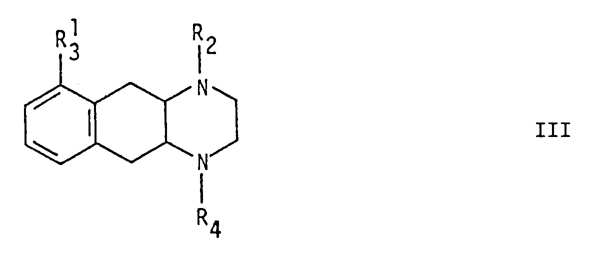 wherein R₂ and R¹₃ are defined as above, and R₄ is a radical which may be reduced to the methyl group,
   are reduced,
and if desired, the compounds of formula I thus obtained are converted into their acid addition salts.

The ether cleavage according to process a) may be effected in known manner, for example using Lewis acids such as boron tribromide or strong mineral acids such as hydrobromic acid.

The alkylation according to process b) may similarly be effected in known manner, e.g. by a reaction with an alkyl halide. There may also be two stages, whereby first of all the compounds of formula II are acylated and then they are reduced.

The reduction according to process c) may be carried out by known methods. R₄ signifies for example an alkoxycarbonyl or menthyloxycarbonyl group.

The enantiomer separation, if desired, may take place by known methods e.g. at the stage of the compounds of formula II or III. If R₁ is methyl, then introduction of the methyl group and splitting of the racemate may be carried out for example simultaneously, whereby in a compound of formula III, wherein R₄ signifies a menthyloxycarbonyl group, after separation of the diastereoisomers, the menthyloxycarbonyl radical is reduced to the methyl group, as described in example 1.

Working up of the reaction mixtures obtained and purification of the compounds of formula I thus produced may be carried out in accordance with known methods.

Acid addition salts may be produced from the free bases in known manner, and vice versa.

6-Hydroxy and 6-alkoxy-1,2,3,4,4a,5,10,10a-octahydro-benzo[g]quinoxalines not covered by formula I are obtained analogously to the preparation of the compounds of formula I.

The starting compounds of formula II and III may be produced by known methods from compounds of formula IX [preparation analogous to Bull. Chem. Soc. Jap. 46, 985 (1973)] in accordance with the following reaction scheme, for example as described in example 1: 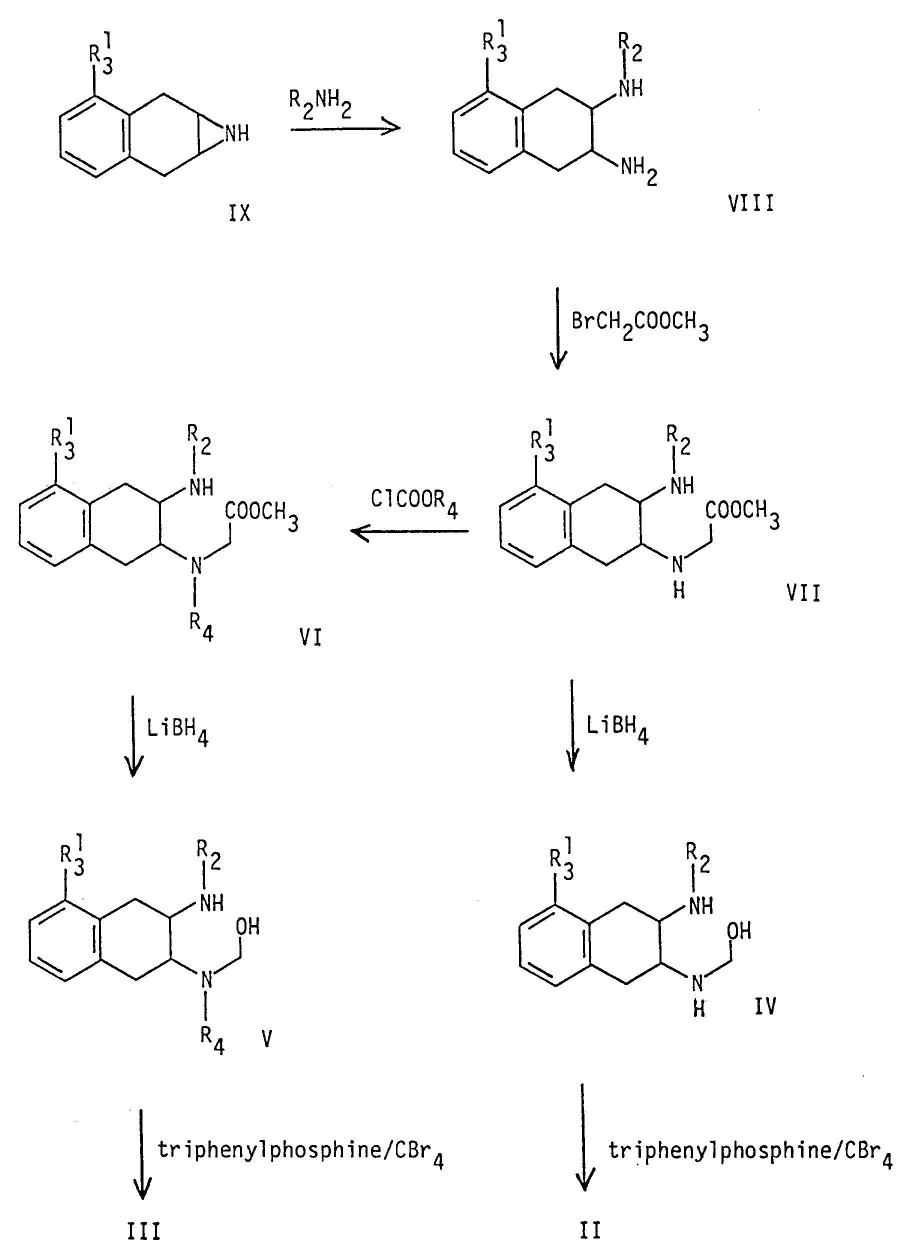
The starting compounds of formula IX are known or may be produced by known processes.

The 6-hydroxy or 6-alkoxy-1,2,3,4,4a,5,10,10a-octahydro-benzo[g]quinoxalines bearing in position 4 an optionally substituted phenyl group and their physiologically acceptable acid addition salts, hereinafter referred to as compounds according to the invention, have interesting pharmacological properties when tested on animals, and may therefore be used as medicaments.

The compounds according to the invention exhibit a selective dopamine antagonistic activity at receptors of the D-1 type (D-1 antagonistic activity), as evidenced by the following experimental data:

At concentrations of ca. 1 nmol/l to ca. 1 µmol/l, the compounds according to the invention effect strong inhibition of the dopamine-induced stimulation of adenylate cyclase in homogenates of bovine retina [for the method see R. Markstein, Journal Neural. Transmission 51, 39 - 59 (1981)].

When administered to the mouse at doses of ca. 0.05 to ca. 1 mg/kg p.o., there is observed a significant inhibition of the rearing induced by subcutaneous administration of 0.5 mg/kg of apomorphine. In this test, each mouse is contained in a glas cylinder (20 cm high, 8 cm diameter) on absorbant cardboard and rearing is quantified by accumulating the time spent with both forepaws off the ground during three 1-minute periods at 10 minutes intervals after apomorphine administration. Test drugs are given 30 minutes before apomorphine.

At doses of ca. 1 to ca. 10 mg/kg p.o., the compounds according to the invention effect no catalepsy of the rat. Cataleptogenic activity is assessed as described by G. Stille et al. in Arzneimittel Forschung 15, 841 - 843 (1965). The criterion for catalepsy is fulfilled when posture retention time is longer than 10 seconds. In view of the lack of cataleptogenic activity, the risk that the compounds according to the invention might cause extra-pyramidal side effects is improbable.

The compounds according to the invention are therefore indicated for the treatment of schizophrenia or of drug abuse (especially cocain abuse) or to antagonize the drug activity in case of a relapse, and also for the treatment of self-injurious behaviour.

An indicated daily dosage is in the range from ca. 1 to 100 mg of the compound according to the invention, and is conveniently administered e.g. in divided doses up to 4 times daily.

The compounds according to the invention may be administered by any conventional route, preferably orally, for example in the form of tablets or capsules, or parenterally, for example in the form of injectable solutions or suspensions.

In accordance with the foregoing, the present invention also provides a compound according to the invention, for use as a pharmaceutical, e.g. for the treatment of schizophrenia, drug abuse or self-injurious behaviour.

The present invention furthermore provides a pharmaceutical composition comprising a compound according to the invention in association with at least one pharmaceutical carrier or diluent. Such compositions may be manufactured in conventional manner. Unit dosage forms contain, for example, from about 0.25 mg to about 50 mg of a compound according to this invention.

In still a further aspect the present invention provides the use of a compound according to the invention for the manufacture of a medicament for the treatment of schizophrenia, drug abuse or self-injurious behaviour.

The following examples illustrate the invention. The temperatures are given in degrees Celsius and are uncorrected.

### EXAMPLE 1: (-)-[4aR,10aR]-1,2,3,4,4a,5,10,10a-octahydro-4-(4'-chloro-2'-methyl-phenyl)-6-methoxy-1-methyl-benzo[g]quinoxaline

**a) trans-2-amino-3-(5'-chloro-2'-toluyl-amino)-5-methoxy-tetralin**
   1.1 ml (8.8 mM) of boron trifluoride ethyl etherate are added in drops at 0° to a solution of 1.5 g (8.5 mM) of 2,3-imino-5-methoxy-tetralin and 1.6 g (11.3 mM) of 4-chloro-2-methylaniline in 40 ml of 2-propanol. After stirring for 20 hours at room temperature, the solution is poured onto ice/water, neutralized with potassium bicarbonate solution, extracted with toluene/ethyl acetate 1:1 and washed with sodium chloride solution. The organic phases are dried over Na₂SO₄, filtered and concentrated by evaporation. 2.7 g of diamine are obtained, and are chromatographed on silica gel with ethyl acetate/ethanol 97:3, containing 0.01% conc. NH₃. The yield is 1.2 g of the desired regio-isomers (title compound).
   NMR (CDCl₃, 360 MHz) δ 2.13 (s, 3H), 2.37 (dd, J₁ = 18 Hz, J₂ = 8 Hz, 1H), 2.76 (dd, J₁ = 18 Hz, J₂ = 11 Hz, 1H), 3.42 (dd, J₁ = 18 Hz, J₂ = 6 Hz, 1H), 3.79 (s, 3H), 6.71 (m, 3H), 7.06 (m, 2H), 7.13 (t, J = 7 Hz, 1H).
**b) trans-2-methoxycarbonylmethylamino-3-(5'-chloro-2'-toluyl-amino)-5-methoxy-tetralin**
   522 mg (3.4 mM) of bromoacetic acid methyl ester in 20 ml of DMF are added at 0° to a suspension of 1.08 g (3.4 mM) of trans-2-amino-3-(5'-chloro-2'-toluyl-amino)-5-methoxy-tetralin and 1.5 g of K₂CO₃, and subsequently stirred for 2 hours at room temperature. Working up is effected by filtering through Hyflo and evaporating the filtrate to dryness. The 1.77 g of raw product are chromatographed on silica gel with hexane/ethyl acetate 2:1. The yield is 0.52 g of the title compound, which is further used directly.
**c)** 76 ml (304 mM) of 4 N NaOH are added in drops at 0° to a solution of 118 g (304 mM) of trans-2-methoxycarbonylmethylamino-3-(5'-chloro-2'-toluyl-amino)-5-methoxy-tetralin and 64.5 ml (304 mM) of chloroformic acid-(-)-menthylester in toluene/2-propanol 10:1. After stirring for 2 hours at room temperature, saturated NaCl solution is added, and the solution is extracted with ethyl acetate. The organic phases are dried over Na₂SO₄, filtered and concentrated. The resulting 180 g of raw product are chromatographed on silica gel with diethylether/hexane 3:7, whereby 119.5 g of pure diasteroisomeric mixture of formula VI are obtained.
**d)** A solution of 117 g (205 mM) of the diastereoisomeric ester of formula VI in ½ litre of THF is added in drops whilst cooling with ice, over the course of 30 minutes, to 15 g (689 mM) of lithium borohydride in 1 litre of THF. After stirring for 16 hours at room temperature, the preparation is cooled again to 0°, and 200 ml of ice water are slowly added. After extracting with ethyl acetate, washing with saturated NaCl solution, drying over Na₂SO₄, filtering and concentrating by evaporation, 104 g (94% of theory) of the diastereoisomeric alcohols of formula V are obtained as a yellowish foam. The raw diastereoisomeric mixture is cyclized directly.
**e) (-)-[4aR,10aR]-1,2,3,4,4a,5,10,10a-octahydro-4-(4'-chloro-2'-methyl-phenyl)-1-(-)-menthyloxycarbonyl-6-methoxybenzo[g]quinoxaline**
   92.5 ml of tetrabromomethane (42.5% in acetonitrile, 119.25 mM) are added in drops at 50°, over the course of 30 minutes, to a solution of 25 g (47.7 mM) of the diastereoisomeric alcohols of formula V and 31 g (119.25 mM) of triphenylphosphine in 250 ml each of toluene and acetonitrile. After stirring for 1 hour at room temperature, the solution is poured onto KHCO₃ solution, extracted with toluene/ethyl acetate 1:1, containing 10% isopropanol, and washed with saturated NaCl solution. The organic phases are dried over Na₂SO₄, filtered and concentrated by evaporation. The raw product is taken up in ethyl acetate and purified preliminarily by a short column of silica gel. After chromatography on SiO₂ with hexane/ether 95:5, 12 g of pure title compound are obtained.
   NMR (DMSO, 360 MHz) δ 0.79 (d, J = 6 Hz, 3H), 0.87 (d, J = 6 Hz, 3H), 0.9 (d, J = 6 Hz, 3H), 1.64 (m, 2H), 1.91 (m, 3H), 2.27 (s, 3H), 2.71 (m, 1H), 2.81 (dd, J₁ = 18 Hz, J₂ = 6 Hz, 1H), 2.96 (dd, J₁ = 18 Hz, J₂ = 12 Hz, 1H), 3.20 (m, 1H), 3.65 (s, 3H), 4.57 (dd, J₁ = 12 Hz, J₂ = 5 Hz, 1H), 6.72 (d, J = 8 Hz, 2H), 7.10 (t, J = 9 Hz, 1H), 7.26 (m, 1H), 7.33 (m, 2H).
**f) (-)-[4aR,10aR]-1,2,3,4,4a,5,10,10a-octahydro-4-(4'-chloro-2'-methyl-phenyl)-6-methoxy-1-methyl-benzo[g]quinoxaline**
   200 ml (200 mM) of diisobutyl aluminium hydride solution (20% in hexane) are added in drops at 0°, over the course of 15 minutes, to a solution, which is under argon, consisting of 16 g (30.5 mM) of (-)-[4aR,10aR]-1,2,3,4,4a,5,10,10a-octahydro-4-(4'-chloro-2'-methyl-phenyl)-1-(-)-menthyloxycarbonyl-6-methoxy-benzo[g]quinoxaline in 200 ml of THF. After stirring for 2 hours at room temperature, it is cooled to 0° and slowly mixed with 75 ml of H₂O, then diluted with 200 ml of ethyl acetate and filtered through Hyflo. The residue of filtration is washed with toluene/ethyl acetate 1:1, containing 10% isopropanol. The filtrate is mixed with saturated NaCl solution and extracted with ethyl acetate. The combined organic phases are dried over Na₂SO₄, filtered and concentrated. The raw product is chromatographed on silica gel with ether/hexane 1:1. 9.4 g of pure title compound are obtained as a white amorphous powder.
   [α]_{D}²⁵ = -11° (pyridine, c = 0.31).
   NMR (DMSO, 360 MHz) δ 1.88 (dd, J₁ = 18 Hz, J₂ = 12 Hz, 1H), 2.10 (m, 1H), 2.29 (s, 3H), 2.32 (s, 3H), 2.45 (m, 1H), 2.61 (dd, J₁ = 18 Hz, J₂ = 12 Hz, 1H), 3.12 (m, 1H), 3.20 (dd, J₁ = 18 Hz, J₂ = 5 Hz, 1H), 3.62 (s, 3H), 6.68 (d, J = 9 Hz, 1H), 6.72 (d, J = 9 Hz, 1H), 7.07 (t, J = 9 Hz, 1H), 7.22 (dd, J₁ = 9 Hz, J₂ = 2 Hz, 1H), 7.28 (d, J = 9 Hz, 1H), 7.34 (d, J = 2 Hz, 1H).

### EXAMPLE 2: (-)-[4aR,10aR]-1,2,3,4,4a,5,10,10a-octahydro-4-(4'-chloro-2'-methyl-phenyl)-1-methyl-benzo[g]quinoxaline-6-ol

49.3 ml (512 mM) of BBr₃ are added in drops at 0°, over the course of 30 minutes, to a solution of 11.4 g (32 mM) of the compound produced in example 1 in 500 ml of CHCl₃. The yellowish suspension is stirred for 2 hours at room temperature. The reaction mixture is subsequently cooled to 0° again and adjusted to pH 8 with 70 ml of NH₃ solution (25%). Extraction with ethyl acetate, containing 10% isopropanol, drying over Na₂SO₄, filtration and concentration, yield 11.3 g of raw product. In order to produce the salt, the base is dissolved in isopropanol and methanol, mixed with 1 equivalent of 3.4 N HCl in EtOH and concentrated until crystallization commences. 9.1 g of pure hydrochloride of the title compound are obtained.
M.p.: from 270° decomp.
[α]_{D}²⁵ = -60° (c = 0.24 in EtOH/H₂O 1:1)
NMR (DMSO, 360 MHz) δ 1.95 (dd, J₁ = 18 Hz, J₂ = 11 Hz, 1H), 2.33 (s, 3H), 2.80 (dd, J₁ = 18 Hz, J₂ = 5 Hz, 1H), 2.95 (d, J = 4 Hz, 3H), 6.61 (d, J = 6 Hz, 2H), 6.96 (t, J = 9 Hz, 1H), 7.30 (s, 2H), 7.40 (s, 1H), 9.44 (s, 1H), 11.21 (b, 1H).

The following compounds wherein R₁ is methyl, R₃ is hydroxy, R₂ is a group of formula (a) as defined above and the configuration is (4aR,10aR) may be produced analogously to example 2:

| Ex. | R'₁ | R'₂ | R'₃ | [α]²⁰_{D} | m.p. | salt |
|---|---|---|---|---|---|---|
| 3 | H | H | H | -127° (1) | (216-218° | bis(base)tartrate |
| 4 | CH₃ | H | H | -73° (2) | from 280° decomp. | methanesulfonate |
| 5 | CH₃ | CH₃ | H | -66° (3) | 291° decomp. | hydrochloride |
| 6 | CH₃ | Br | H | -44° (4) | 260° decomp. | hydrochloride |
| 7 | CH₃ | CH₃ | CH₃ | -58° (5) | 270° decomp. | hydrochloride |
| 8 | CH₃ | F | H | -57° (6) | 250° decomp. | hydrochloride |

| | | | | | | |
|---|---|---|---|---|---|---|
| (1) pyridine, c = 0.15 | | | | | | |
| (2) EtOH/H₂O 1:1, c = 0.215 | | | | | | |
| (3) EtOH/H₂O 1:1, c = 0.325 | | | | | | |
| (4) EtOH/H₂O 1:1, c = 0.19 | | | | | | |
| (5) EtOH/H₂O 1:1, c = 0.275 | | | | | | |
| (6) EtOH/H₂O 1:1, c = 0.265 | | | | | | |

### EXAMPLE 9: (±)-[4aα,10aβ]-1,2,3,4,4a,5,10,10a-octahydro-6-methoxy-4-phenyl-1-n-propyl-benzo[g]quinoxaline

1 g K₂CO₃ and 360 µl (about 3.6 mM) n-propyliodide are added to 900 mg (about 3 mM) (±)-[4aα,10aβ]-1,2,3,4,4a,5,10,10a-octahydro-6-methoxy-4-phenyl-benzo[g]quinoxaline in 30 ml dimethylformamide and the reaction mixture is stirred for 42 hours at room temperature. Working up is effected by filtering through Hyflo and evaporating the filtrate to dryness. The residue is taken up in methylenechloride containing 10 % isopropanol and extracted with concentrated NaCl solution. The combined organic phases are dried over Na₂SO₄, filtered and concentrated. One obtains 1 g of the crude title compound. M.p. of the dihydrochloride: 236 - 238 °.

## Claims

1. A 6-hydroxy or 6-alkoxy-1,2,3,4,4a,5,10,10a-octahydro-benzo[g]quinoxaline bearing in position 4 an optionally substituted phenyl group, in free base or acid addition salt form.

2. A compound of formula I 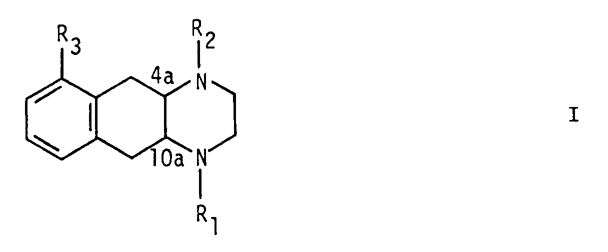 wherein
R₁ is (C₁₋₄)alkyl,
R₂ is phenyl optionally mono-, di- or trisubstituted by halogen with an atomic number of 9 to 53, (C₁₋₄)alkyl and/or (C₁₋₄)alkoxy, and
R₃ is hydroxy or (C₁₋₄)alkoxy,
in free base or acid addition salt form.

3. A compound of claim 2 wherein R₂ is a group of formula (a) 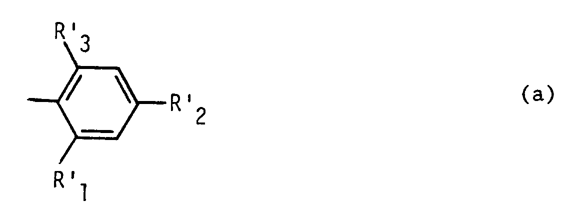 wherein
R'₁ is hydrogen or (C₁₋₄)alkyl,
R'₂ is hydrogen, halogen with an atomic number of 9 to 53 or (C₁₋₄)alkyl, and
R'₃ is hydrogen or (C₁₋₄)alkyl,
in free base or acid addition salt form.

4. The (-)-[4aR,10aR]-1,2,3,4,4a,5,10,10a-octahydro-4-(4'-chloro-2'-methyl-phenyl)-1-methyl-benzo[g]quinoxaline-6-ol, in free base or acid addition salt form.

5. A compound of claim 2 wherein
- R₁ = CH₃, R₂ = 4-Cl, 2-CH₃-phenyl, R₃ = OCH₃
- R₁ = CH₃, R₂ = phenyl, R₃ = OH
- R₁ = CH₃, R₂ = 2-CH₃-phenyl, R₃ = OH
- R₁ = CH₃, R₂ = 2,4-diCH₃-phenyl, R₃ = OH
- R₁ = CH₃, R₂ = 4-Br, 2-CH₃-phenyl, R₃ = OH
- R₁ = CH₃, R₂ = 2,4,6-triCH₃-phenyl, R₃ = OH
- R₁ = CH₃, R₂ = 4-F, 2-CH₃-phenyl, R₃ = OH
- R₁ = n-C₃H₇, R₂ = phenyl, R₃ = OCH₃
in free base or acid addition salt form.

6. A compound of any one of claims 1 to 5 in free base or physiologically acceptable acid addition salt form, for use as a pharmaceutical.

7. A compound of any one of claims 1 to 5 in free base or physiologically acceptable acid addition salt form, for use in the treatment of schizophrenia, drug abuse or self-injurious behaviour.

8. A pharmaceutical composition comprising a compound of any one of claims 1 to 5 in free base or physiologically acceptable acid addition salt form, in association with a pharmaceutical carrier or diluent.

9. The use of a compound of any one of claims 1 to 5 in free base or physiologically acceptable acid addition salt form, for the manufacture of a medicament for the treatment of schizophrenia, drug abuse or self-injurious behaviour.

10. A process for the production of compounds of formula I as defined in claim 2, whereby
a) in order to produce the compounds of formula Ia, 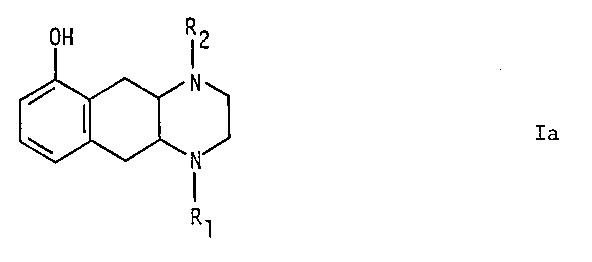 wherein R₁ and R₂ are as defined in claim 2, the ether group in compounds of formula Ib, 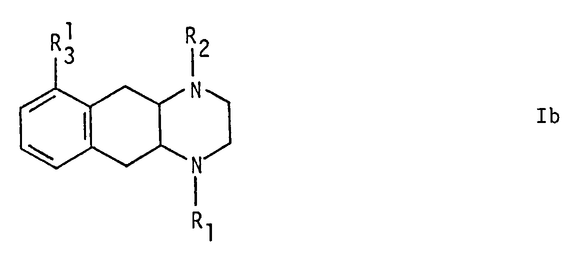 wherein R₁ and R₂ are as defined in claim 2, and R¹₃ is alkoxy with 1 to 4 carbon atoms, is cleaved, or
b) in order to produce compounds of formula Ib, compounds of formula II, 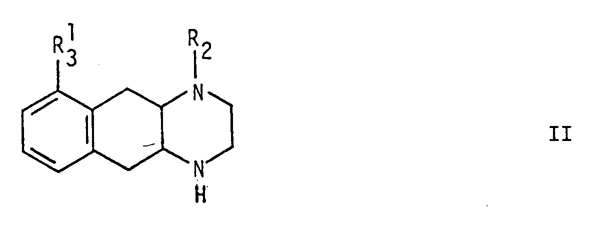 wherein R₂ and R¹₃ are defined as above, are alkylated, or
c) in order to produce compounds of formula I'b, 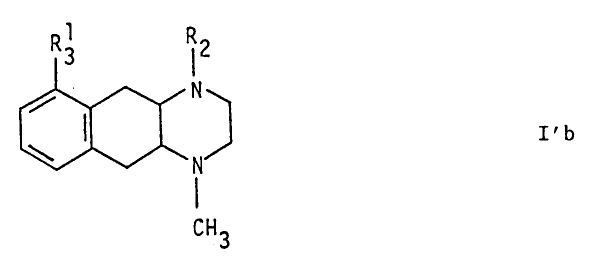 wherein R₂ and R¹₃ are defined as above, compounds of formula III, 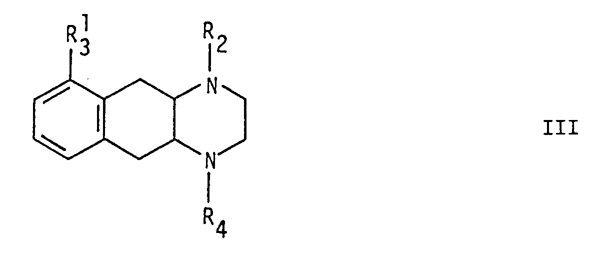 wherein R₂ and R¹₃ are defined as above, and R₄ is a radical which may be reduced to the methyl group, are reduced,
and if desired, the compounds of formula I thus obtained are converted into their acid addition salts.
